# EUROPEAN PATENT APPLICATION

(11) **EP 0 891 772 A2**
(43) Date of publication of application: **20.01.1999**
(21) Application number: 98110865.7
(22) Date of filing: 15.06.1998
(51) Int. Cl.: A61K 31/195, A61K 31/19

(54) **Therapeutic agent for rheumatic disease comprising nonsteroidal anti-inflamatory drug and phenylpropionic acid derivative**

(30) Priority: 18.06.1997 JP 160801/97
(71) Applicant: SANTEN PHARMACEUTICAL CO., LTD., Higashiyodogawa-ku, Osaka-shi, Osaka 533-8651 (JP); ONO PHARMACEUTICAL CO., LTD., Osaka-shi, Osaka 541-8526 (JP)
(72) Inventor: Inukai, Toshiya, Santen Pharmaceutical Co., Ltd., Osaka-shi, Osaka 533-8651 (JP); Matsuno, Kiyoshi, Santen Pharmaceutical Co., Ltd., Osaka-shi, Osaka 533-8651 (JP); Fujisawa, Koushi, Santen Pharmaceutical Co., Ltd., Osaka-shi, Osaka 533-8651 (JP); Murai, Masaaki, Santen Pharmaceutical Co., Ltd., Osaka-shi, Osaka 533-8651 (JP); Omawari, Nagashige, Ono Pharmaceutical Co., Ltd., Mishima-gun, Osaka 618-8585 (JP); Terai, Koichiro, Ono Pharmaceutical Co., Ltd., Mishima-gun, Osaka 618-8585 (JP)
(74) Representative: Paul, Dieter-Alfred, Dipl.-Ing.

(57) **Abstract**

An object of the present invention is to find a novel usefulness of 3- [1- [6- (4-methoxyphenyl) hex-5E-enyl] oxy-3- (4-carboxybutyl) oxybenzen-2-yl]propionic acid. The present invention relates to a therapeutic agent for rheumatic disease comprising a combination of a nonsteroidal anti-inflammatory drug with 3-[1-[6-(4-methoxyphenyl)hex-5E-enyl]oxy-3-(4-carboxybutyl)oxybenzen-2-yl]propionic acid or a salt thereof. Both components in this combination complement and/or potentiate their actions each other. The nonsteroidal anti-inflammatory drug is preferably diclofenac or a salt thereof.

## Description

### Background of the Invention

The present invention relates to a therapeutic agent for rheumatic disease comprising a combination of a nonsteroidal anti-inflammatory drug and 3-[1-[6-(4-methoxyphenyl)hex-5E-enyl]oxy-3-(4-carboxybutyl) oxybenzen-2-yl]propionic acid or a salt thereof.

Rheumatic disease is an intractable chronic inflammatory disease where arthrosynovial membrane is a main site of lesion but there are still many ambiguous points for its onset cause and there are various symptoms as well. One of the characteristics of pathology of the rheumatic disease is abnormal multiplication of synovial membrane and the succeeding destruction of cartilage and bone. Therapy of rheumatic disease is mainly conducted by means of a pharmacotherapy and, with regard to the drugs therefor, steroidal agents and nonsteroidal anti-inflammatory drugs have been used for curing the inflammation in rheumatic disease. When it has been known recently that immunity participates in rheumatic disease, immunosuppressive agent, immunomodulators (DMARDs), etc. have been used. In addition, study of combination of various drugs has been tried as well with an object of complimenting the disadvantage and of making the best use of advantage of each of the drugs.

Recently, studies of drugs based upon new function mechanism have been actively conducted as well. A leukotriene B₄ (LTB₄) antagonist is one of them. It has been known that the LTB₄, which is a metabolite in a 5-lipoxygenase system orginating from arachidonic acid, plays an important role in the function of leukocytes and has an action of inducing the inflammation. To be more specific, it has a strong ability of migration, taxis, aggregation and active oxygen production and release to leukocytes and, in addition, it acts the hemangioendothelium whereby adhering ability of leukocytes to hemangioendothelial cells is accelerated. On the other hand, it has been reported that the LTB₄ increases in articular liquid of patients suffering from rheumatic disease and promotes the production of interleukin-1 in cultured synovial membrane cells obtained from the patients with rheumatic disease (Rhumachi, 31, 762(1991)). There is another paper reporting that, when the amount of LTB₄ is reduced, rheumatic disease, inflammatory bowel diseases, etc. can be improved (Exp. Opin. Invest. Drugs, 5, 73-77(1996)).

It is disclosed in Examined Japanese Patent Publication No. 7-39,369 that 3-[1-[6-(4-methoxyphenyl)hex-5E-enyl]oxy-3-(4-carboxylbutyl)oxybenzen-2-yl]propionic acid (hereinafter, this will be referred to as "the present compound") represented by the following formula [I] has an antagonism to LTB₄ and is useful for prevention and/or therapy of various diseases such as allergic dermatitis, rheumatic disease, gout, psoriasis, arthritis, trichophytosis and myocardial infarction. It has been also reported that the present compound competitively inhibits the receptor binding of LTB₄ to human neutrophils (Prostaglandins, 44, 261-275(1982)) and shows an immunosuppressive action to hepatic allotransplantation in rats (Surg. Today, 26, 419-426(1996)).

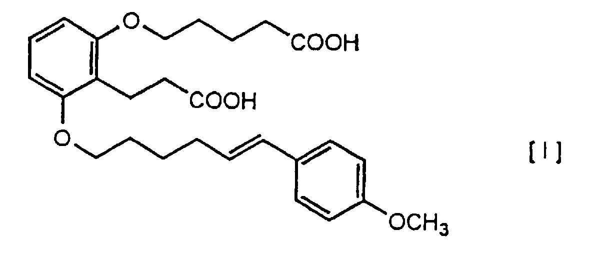

In addition, there have been various studies on combination of LTB₄ receptor antagonist with other drugs as well. For example, an effect of combination of cycloalkylcarboxylic acid derivatives with nonsteroidal anti-inflammatory drugs on collagen-induced arthritis (Arthritis Rheum., 39, 515-521)1996)), an effect of combination of benzopyran derivatives with cyclooxygenase-2 inhibitor on collagen-induced arthritis (WO96/41645) and an effect of combination of pyridine-substituted benzyl alcohol derivatives with nonsteroidal anti-inflammatory drugs on asthma rats of a mating type (Japanese National Publication of Translation of PCT (KOHYO) No. 7-505,401) have been reported.

However, there has been no report at all on the effect of combination of the present compound with a nonsteroidal anti-inflammatory drug.

As such, it is a very interesting matter to find a new usefulness by combination of the present compound (which exhibits not only an LTB₄ receptor antagonistic action but also an immunosuppressive action) with a nonsteroidal anti-inflammatory drug.

### Summary of the Invention

The present inventors focused on treatment of rheumatic disease and precisely studied the effect of combination of a nonsteroidal anti-inflammatory drug the present compound. Various methods for investigating the effect on rheumatic disease using animal models have been reported already. They are, for example, a method using a collagen-induced arthritis model (Nature, 283, 666-668(1980); Proc. Natl. Acad. Sci. USA., 92, 517-521)1995)), a method using an adjuvant-induced arthritis model (Ann. Rheum. Dis., 15, 379-380(1956); Br. J. Pharmacol., 21, 127-136(1963)), etc. Since rheumatic disease is accompanied by inflammation and edema of joints, a method of investigation using a zymosan-induced edema model as an acute inflammation model has been known as well (Agents Actions, 32, 119-121(1991)). Accordingly, in the present invention, an effect on rheumatic disease was investigated using those animal models. As will be given in the detailed test method and the result thereof which will be mentioned in Examples (under the item of pharmacological test) later, combination of a nonsteroidal anti-inflammatory drug with the present compound showed a significant suppressive effect on such models. In addition, the instant effect is synergistic and is quite unexpected from the effect of each of the nonsteroidal anti-inflammatory drug and the present compound. Those resilts clearly show that the combination of the nonsteroidal anti-inflammatory drug with the present compound is very useful as a therapeutic agent for rheumatic disease. The therapeutic agent according to the present invention can be used effectively for not only treatment but also prevention of rheumatic disease.

### Brief Description of the Drawings

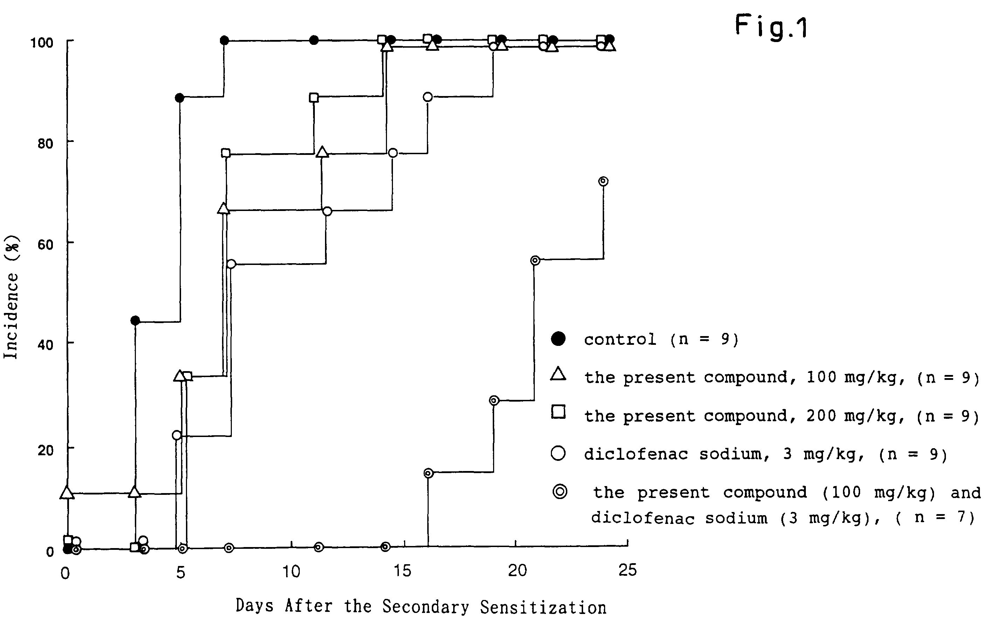

Fig. 1 is a graph showing the daily changes of incidence of arthritis in type II collagen-induced arthritic mice. In the graph, ⓞ shows the result (average of seven cases) in a group where 3-[1-[6-(4-methoxyphenyl)hex-5E-enyl]oxy-3- (4-carboxybutyl)oxybenzen-2-yl]propionic acid (the present compound) (100 mg/kg) and diclofenac sodium (3 mg/kg) were administered in combination. △ and □ show the results (average of nine cases in each dose) in a group where the present compound alone was administered (100 mg/kg and 200 mg/kg, respectively). ○ shows the result (average of nine cases) in a group where diclofenac sodium alone was administered (3 mg/kg). ● shows the result (average of nine cases) in a group where no test compound was administered (a control group).

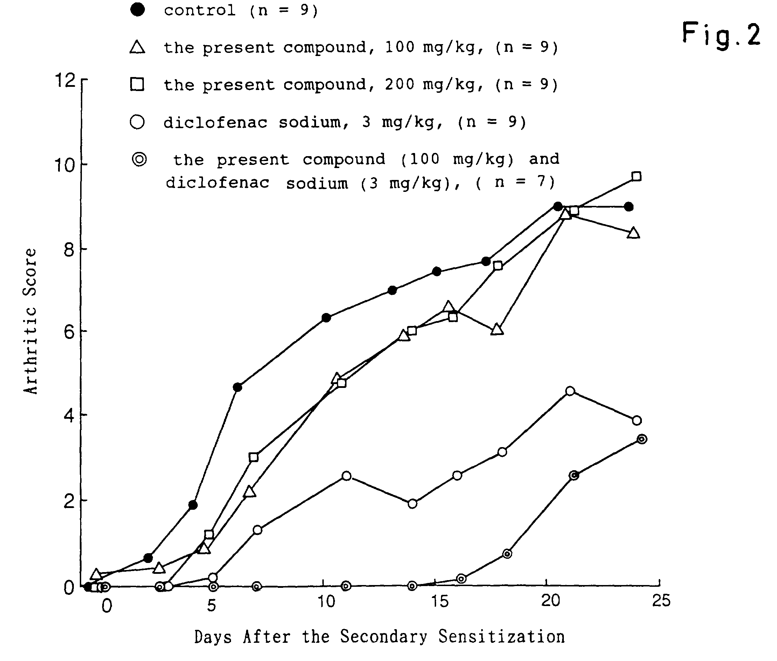

Fig. 2 is a graph which shows daily changes in arthritic scores in type II collagen-induced arthritic mice. In the graph, ⓞ shows the result (average of seven cases) in a group where the present compound (100 mg/kg) and diclofenac sodium (3 mg/kg) were administered in combination. △ and □ show the results (average of nine cases in each dose) in a group where the present compound alone was administered (100 mg/kg and 200 mg/kg, respectively). ○ shows the result (average of nine cases) in a group where diclofenac sodium alone was administered (3 mg/kg). ● shows the result (average of nine cases) in a group where no test compound was administered (a control group).

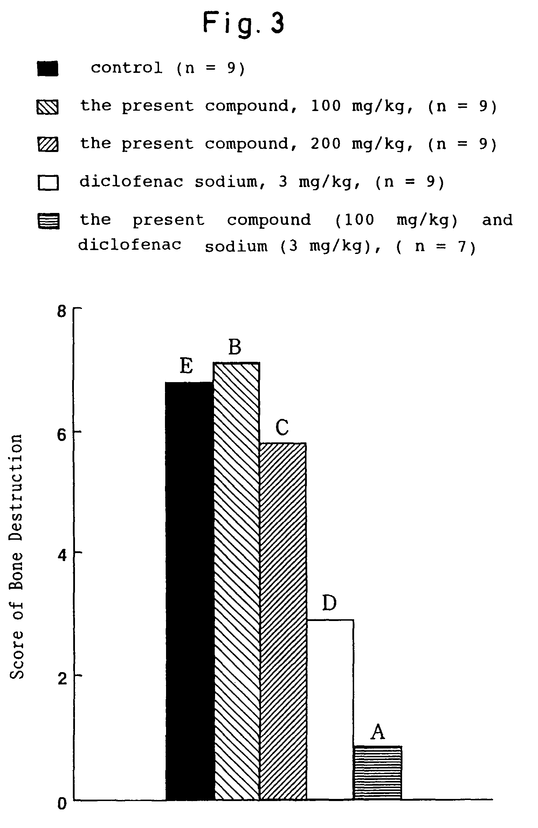

Fig. 3 is a graph showing a score of bone destruction in arthritis in type II collagen-induced arthritic mice. In the graph, A shows the result (average of seven cases) in a group where the present compound (100 mg/kg) and diclofenac sodium (3 mg/kg) were administered in combination. B and C show the results (average of nine cases in each dose) in a group where the present compound alone was administered (100 mg/kg and 200 mg/kg, respectively). D shows the result (average of nine cases) in a group where diclofenac sodium alone was administered (3 mg/kg). E shows the result (average of nine cases) in a group where no test compound was administered (a control group).

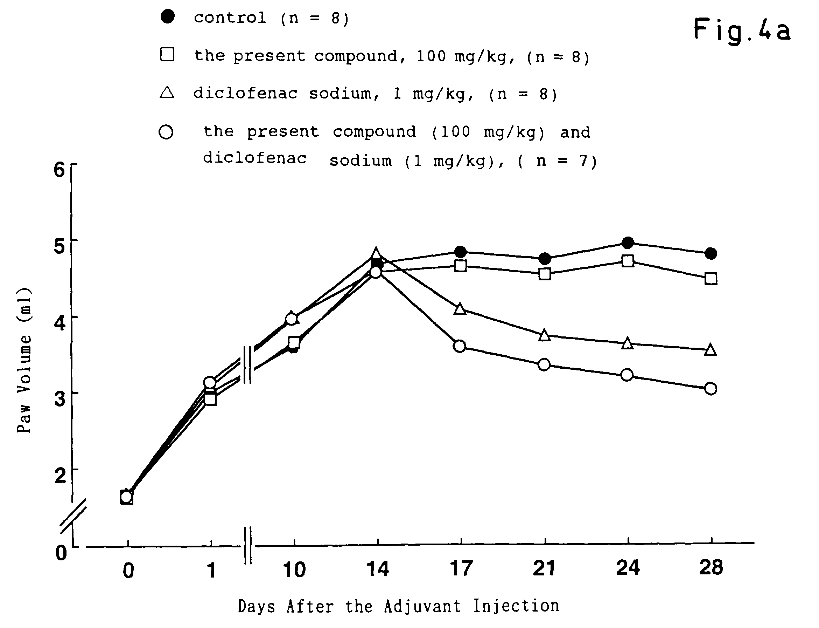

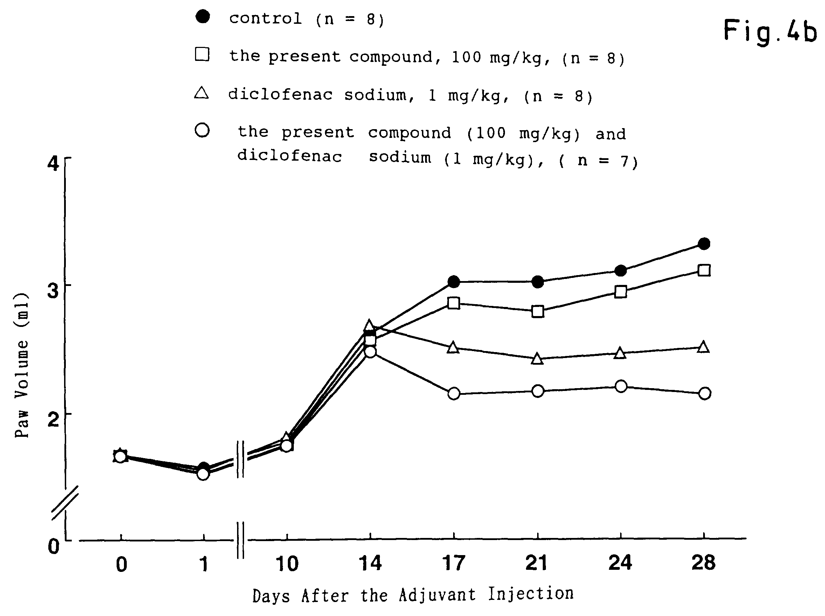

Fig. 4 (a) and (b) are the graphs showing the daily changes in paw volumes in adjuvant-induced arthritic rats. Thus, Fig. 4a shows paw volume of left hindpaw while Fig. 4b shows paw volume of right hindpaw. In the graphs, ○ shows the result in the group where the present compound (100 mg/kg) and diclofenac sodium (1 mg/kg) were administered in combination (average of the seven cases). □ shows the result in the group where the present compound (100 mg/kg) alone was administered (average of the eight cases). △ shows the result in the group where diclofenac sodium (1 mg/kg) alone was administered (average of the eight cases). ● shows the result in the group where no compound to be tested was administered (control group; average of the eight cases).

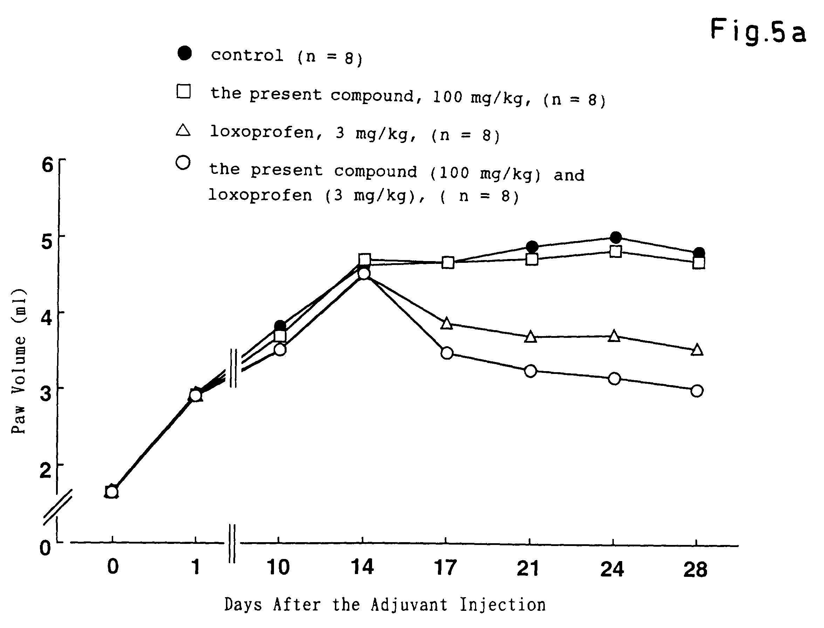

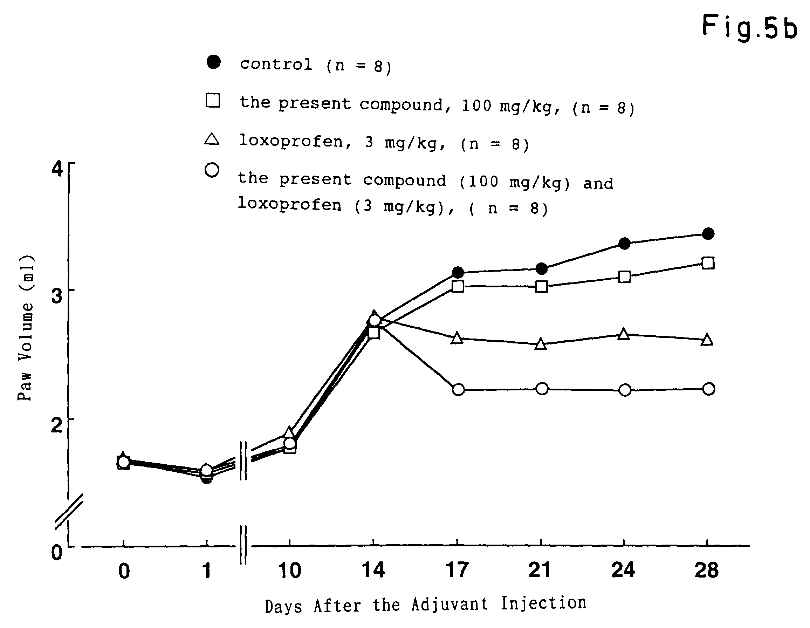

Fig. 5 (a) and (b) are the graphs showing the daily changes in paw volumes in the adjuvant-induced arthritic rats. Thus, Fig. 5a shows paw volume of left hindpaw while Fig. 5b shows paw volume of right hindpaw. In the graphs, ○ shows the result in the group where the present compound (100 mg/kg) and loxoprofen (3 mg/kg) were administered in combination (average of the eight cases). □ shows the result in the group where the present compound (100 mg/kg) alone was administered (average of the eight cases). △ shows the result in the group where loxoprofen (3 mg/kg) alone was administered (average of the eight cases). ● shows the result in the group where no compound to be tested was administered (control group; average of the eight cases).

### Description of the Preferred Embodiments

The present invention relates to a therapeutic agent for rheumatic disease comprising a combination of a nonsteroidal anti-inflammatory drug and 3-[1-[6-(4-methoxyphenyl)hex-5E-enyl]oxy-3-(4-carboxybutyl) oxybenzen-2-yl]propionic acid (the present compound) or a salt thereof and, more particularly, it relates to a therapeutic agent for rheumatic disease which is characterized in that each of their actions is complemented and/or potentiated.

For the therapy of rheumatic disease, the nonsteroidal anti-inflammatory drug and the present compound can be administered in a form in which both are formulated in one preparation or can be administered in a form of separate preparations, i.e. by way of an administration in combination of the preparations.

The present compound can be in a form of a salt. There is no particular limitation for the salt in the present invention so far as it is a pharmaceutically acceptable one and its examples are a salt with an inorganic acid such as hydrochloric acid, nitric acid or sulfuric acid; a salt with an alkali metal or an alkali earth metal such as sodium, potassium or calcium; ammonium salt; and a salt with an organic acid such as diethylamine or triethanolamine. It is also possible that the present compound can be in a form of a hydrate.

The present invention is characterized in that a nonsteroidal anti-inflammatory drug and the present compound are combined to give a therapeutic agent for rheumatic disease and there is no limitation for the type of the nonsteroidal anti-inflammatory drug used. In the present invention, a known nonsteroidal anti-inflammatory drug can be used and its examples are diclofenac, loxoprofen, ketoprofen, ibuprofen, naproxen, indomethacin and piroxicam. Some of them are actually used in a form of salt such as sodium salt but, in this specification, description of "salt" is omitted except when it is specifically referred to. Incidentally, the nonsteroidal anti-inflammatory drug which can be used in the present invention includes ones in a form of a so-called prodrug.

Pharmaceutical preparation for conducting the present invention can be that in which the nonsteroidal anti-inflammatory drug and the present compound are formulated in one preparation and that in which each of them is made into different preparations separately. There is no need of using any special technique for preparing such preparations but widely used technique may be applied for preparing them. Examples of the dosage form are tablets, capsules, powder, granules, injections for topical application, liquid preparations for external use and liniments and the administration can be conducted either by means of systemic or topical administration.

When the nonsteroidal anti-inflammatory drug and the present compound are formulated into separate preparations, that can be prepared according to known methods. For example, the preparation disclosed in Examined Japanese Patent Publication No. 7-39,369 can be used as a preparation of the present compound while, as a preparation of the nonsteroidal anti-inflammatory drug, commercially-available products can be used. In the case of the preparation where the nonsteroidal anti-inflammatory drug and the present compound are formulated, it can also be prepared according to known methods like the above. For example, in the manufacture of solid dosage forms such as tablets, capsules, powder and granules, the preparation can be manufactured using, if necessary, diluents such as lactose, crystalline cellulose and starch; lubricants such as magnesium stearate and talc; binders such as hydroxypropyl cellulose and polyvinylpyrrolidone; disintegrator such as calcium carboxymethyl cellulose and low substituted hydroxypropylmethyl cellulose; and coating agents such as hydroxypropylmethyl cellulose, Macrogol and silicone resin.

For reference, some examples of preparation will be described in Examples (under the item of Preparation Examples) which will be given later but it goes without saying that such Preparation Examples do not limit the scope of the present invention.

Doses of the nonsteroidal anti-inflammatory drug and the present compound will be decided depending upon symptom, age, etc. of the patient and, usually, the present compound is administered once or several times a day where the daily dose is within a range of from 1 to 1,000 mg. Preferably, the daily dose is 10 to 500 mg and, more preferably, it is 50 to 300 mg. Dose of the nonsteroidal anti-inflammatory drug varies depending upon the type of the agent and, usually, it is decided depending on the dose range which is actually used as an anti-inflammatory drug and can be appropriately modified according to the symptom, etc. Its daily dose is within a range of from 1 to 1,000 mg and is administered once or several times a day. A preferred daily dose is within a range of from 10 to 600 mg. To be more specific, daily dose is usually 75 to 100 mg in the case of diclofenac sodium, but the dose can be appropriately modified depending upon the symptom, etc. Doses of other nonsteroidal anti-inflammatory drugs can be decided according to the similar conception as the above. Those doses can also be appropriately modified according to the dosage form. Those doses will be applied to the case where the preparation of the present compound and that of the nonsteroidal anti-inflammatory drug are administered in combination. In the case where the preparation in which the present compound and the nonsteroidal anti-inflammatory drug are formulated in one preparation is administered, the preparation where the formulating ratio of them is suitably selected so that the daily dose is within the above-mentioned range is prepared and the resulting preparation is administered once or several times a day.

As hereunder, Pharmacological Test and Preparation Examples are shown as examples. However, those examples are mentioned here for better understanding of the present invention and are not for limiting the scope of the present invention.

### Examples

### Pharmacological Test:

### (1) Effect on Type II Collagen-Induced Arthritis:

Model of type II collagen-induced arthritis has been widely used as an animal model of rheumatic disease and there is a paper in which the inhibiting effect of LTB4 receptor antagonist using the instant model was studied (Proc. Natl. Acad. Sci. USA., 92, 517-521(1995)).

Now, the effect of the combination use of the present compound and the nonsteroidal anti-inflammatory drug on type II collagen-induced arthritis was investigated according to the method described in the above paper.

### (Experimental Method)

A solution (4 mg/ml) of bovine joint cartilage type II collagen in diluted hydrochloric acid was mixed with Freund' s complete adjuvant of the same volume to prepare an emulsion. As a primary sensitization, this emulsion was injected intra-dermally into tail root site of DBA/1 strain mouse at a dose of 100 µl (corresponding to 200 µg of the type II collagen per mouse). After 21 days from the primary sensitization, 100 µl of this emulsion was injected intra-dermally into a tail root site of the mouse again (secondary sensitization).

A suspension of a compound to be tested which was prepared so as to adjust the dose 10 ml per 1 kg of body weight was orally administered once a day continuously from the day before the primary sensitization, whereby the present compound was suspended in 0. 5% carboxymethyl cellulose and a nonsteroidal anti-inflammatory drug was suspended in 1% methyl cellulose.

With regard to the confirmation of onset of arthritis, the stage where swelling at the site of joint accompanied by erythema was defined as the onset of arthritis. With regard to degree of the arthritis, scoring was conducted for each paw according to the evaluating criterion for arthritis grade as mentioned below and the total point for four paws was used as an arthritic score.

### Evaluating Criterion for Grade of Arthritis:

0: no change
1: weak swelling and weak erythema were observed
2: weak swelling and erythema were observed
3: strong swelling and erythema were observed
4: strong swelling and erythema accompanied by bone erosion were observed

Then, on the 26th day after the secondary sensitization, X-ray photographs of the joints of four paws were taken. Observation under a microscope was conducted and scoring was done for each paw according to the evaluating criterion for grade of bone destruction as shown below and the total point for four paws was used as a bone destruction score.

### Evaluating Criterion for Grade of Bone Destruction:

0: no change
1: bone destruction was observed in one finger
2: bone destruct ion was observed in two or more fingers
3: bone destruction was observed in whole joint

### (Results)

As one example of the experimental results, when the present compound (100 mg/kg) and diclofenac sodium (3 mg/kg), which is a nonsteroidal anti-inflammatory drug, were administered in combination, the incidence of arthritis is shown in Fig. 1; changes in arthritic score is shown in Fig. 2; and bone destruction score is shown in Fig. 3.

For comparison, the results of the administrations of the present compound alone (100 mg/kg and 200 mg/kg), administration of diclofenac sodium alone (3 mg/kg), and non-administration of any test compound (control) are shown in Fig. 1 to Fig. 3 as well.

First, as shown in Fig. 1 and Fig. 2, in the control group, onset of arthritis was observed from the third day after the secondary sensitization and, on the seventh day after the secondary sensitization, onset of arthritis was observed in all of the cases. After that, worsening of the symptom of arthritis continued until the 21st day after the secondary sensitization.

Unlike the above onset of arthritis, in a group where 100 mg/kg of the present compound and 3 mg/kg of diclofenac sodium were used in combination, no onset of arthritis was observed until the 14th day after the secondary sensitization so that a significant effect for delaying the onset of arthritis was recognized. There was a remarkable improvement in the arthritic score as well and, until the 19th day from the secondary sensitization, no strong swelling accompanied by erythema was observed.

However, in a group where 100 mg/kg of the present compound alone was administered, improvement was recognized slightly in arthritic score but the incidence of arthritis showed just nearly the same proceeding as in the control group. Such a tendency was unchanged even when the dose was increased to 200 mg/kg.

In a group where 3 mg/kg of diclofenac sodium alone was administered, improvement in the arthritic score was recognized but no effect of delaying the onset of arthritis was recognized.

Fig. 3 shows the suppressive action to bone destruction and, in a group where 100 mg/kg of the present compound and 3 mg/kg of diclofenac sodium were administered in combination, significant suppressive effect on bone destruction was recognized as compared with a control group. On the contrary, in a group where 100 mg/kg of the present compound alone was administered, no suppressive effect was recognized at all and, even when the dose was increased to 200 mg/kg, the suppressive effect was hardly recognized as well. In a group where 3 mg/kg of diclofenac sodium alone was administered, a suppressive effect was recognized but the instant effect was far weaker than in the case of the combination administration.

From the above result, it is now apparent that the combination use of the present compound and the nonsteroidal anti-inflammatory drug significantly suppresses the type II collagen-induced arthritis and bone destruction and, moreover, the resulting effect is so excellent that it is unable to be expected from the effects of administration of the present compound alone or of the nonsteroidal anti-inflammatory drug alone. (2) Effect on Adjuvant-Induced Arthritis:

Adjuvant-induced arthritis model is a model which has been widely used as a model for investigating the action of drugs to joint. Thus, the effect of the combination use of the present compound and nonsteroidal anti-inflammatory drug was investigated using the instant model.

### (Method of Experiment)

A suspension (0.6 mg/100 µl) of Mycobacterium butyricum (adjuvant) in liquid paraffin was injected subcutaneously into a left hind paw of rat (male; nine weeks age; body weight being about 240 grams) to induce arthritis. Starting from the 14th day after injection of the adjuvant, a suspension of a compound to be tested which was prepared so as to adjust the dose 0.5 ml per 100 g of body weight was orally administered once a day, whereby the present compound was suspended in 0.5% carboxymethyl cellulose and a nonsteroidal anti-inflammatory drug was suspended in 1% methyl cellulose.

Incidentally, as a control, a suspension not containing a test compound was administered by the same manner as above.

### (Results)

When arthritis was induced, edema was observed in paws and the paw volume increased. Then, a left hindpaw volume when 100 mg/kg of the present compound was administered in combination with 1 mg/kg of diclofenac sodium or 3 mg/kg of loxoprofen as a typical example of the nonsteroidal anti-inflammatory drug is shown in Figs. 4a or 5a, respectively. Incidentally, arthritis is expressed systemically and such an expression is observed not only at the site where the adjuvant is injected but also at the right hindpaw. The result of measurement of the right hindpaw volume is shown in Figs. 4b and 5b.

For comparison, the results of administration of the present compound alone, the nonsteroidal anti-inflammatory drug alone and the control group are also shown in each of the corresponding figures.

Further, after measuring the above-mentioned test results, a pathohistological test was conducted too.

As shown in Figs. 4a and 5a, the edema of left hindpaw induced by injection of the adjuvant became almost stationary state on the 14th day. In the group where the present compound and the nonsteroidal anti-inflammatory drug were administered in combination, the edema was significantly suppressed. On the contrary, in the group where the present compound alone was administered, nearly no suppressive effect on edema was recognized. Although a suppressive effect on edema was recognized in the group where the nonsteroidal anti-inflammatory drug alone was administered, the effect by the combination use was significantly better. Further, as shown in Figs. 4b and 5b, edema occurred in the right hindpaw on the tenth day after injection of the adjuvant. When the paw volume was measured, it was similar that in the case of the left hindpaw. Those results clearly show the synergistic effect by the combination use of the present compound and the nonsteroidal anti-inflammatory drug.

Furthermore, in the pathohistological test, there was nearly no suppressive effect on the changes in terms of observation of bone destruction, edema, etc. in a group where the present compound alone or the nonsteroidal anti-inflammatory drug alone was administered, while in the group where the present compound and the nonsteroidal anti-inflammatory drug were administered in combination, an effect was apparently achieved.

### (3) Effect on Zymosan-Induced Paw Edema:

Rheumatic disease is a disease accompanied by inflammation and, as an animal model for testing the suppressive effect of LTB₄ receptor antagonist on such inflammation, a zymosan induced paw edema model has been reported (Agents Actions, 32, 119-121 (1991)).

Accordingly, the effect of the combination use of the present compound and the nonsteroidal anti-inflammatory drug on zymosan-induced paw edema was studied according to the method described in the above-mentioned literature reference.

The experiment was conducted in accordance with the following method.

A suspension (2%) of zymosan in saline was injected subcutaneously at a dose of 100 µl into a left hindpaw of rat to induce paw edema and the volume of the left hindpaw was measured by a plethysmometer immediately before the injection and after a certain period after the injection of zymosan. The compound to be tested was orally administered one hour before the injection of zymosan.

An effect of the test compound was judged using the change to the paw volume immediately before injection of zymosan as an index.

As a result, in a group where the present compound and the nonsteroidal anti-inflammatory drug are administered in combination, a remarkable suppressing effect on edema was recognized as compared with the group where the present compound or the nonsteroidal anti-inflammatory drug alone was administered.

### [Preparation Examples]

The following components were mixed by a conventional method and filled in capsules.

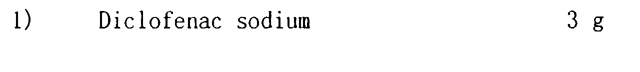

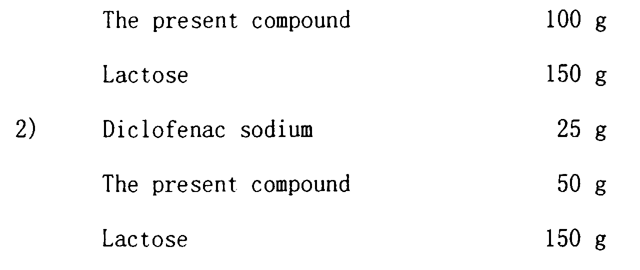

When the combination ratio of the components are changed, desired capsule preparations can be prepared accordingly. It is also possible to use other nonsteroidal anti-inflammatory drug instead of diclofenac sodium for preparing the similar capsule preparations.

From the results of the above pharmacological tests, it has now been found that the combination of a nonsteroidal anti-inflammatory drug with the present compound or a salt thereof is very useful in a therapy for rheumatic disease.

## Claims

1. A therapeutic agent for rheumatic disease comprising a nonsteroidal anti-inflammatory drug and 3-[1-[6-(4-methoxyphenyl)hex-5E-enyl]oxy-3-(4-carboxybutyl)oxybenzen-2-yl]propionic acid or a salt thereof.

2. A therapeutic agent for rheumatic disease comprising a nonsteroidal anti-inflammatory drug and 3-[1-[6-(4-methoxyphenyl)hex-5E-enyl]oxy-3-(4-carboxybutyl)oxybenzen-2-yl]propionic acid or a salt thereof, wherein their actions are complemented and/or potentiated each other.

3. A therapeutic agent for rheumatic disease according to claim 1 or 2, wherein the nonsteroidal anti-inflammatory drug is diclofenac or a salt thereof.
